# EUROPÄISCHE PATENTANMELDUNG

(11) **EP 4 144 504 A1**
(43) Veröffentlichungstag der Anmeldung: **08.03.2023**
(21) Anmeldenummer: 22202325.1
(22) Anmeldetag: 20.12.2013
(51) Int. Cl.: B29C 49/42, B08B 9/28, B08B 9/34

(54) **VORRICHTUNG UND VERFAHREN ZUM STERILISIEREN VON BEHÄLTNISSEN**

(30) Priorität: 20.12.2012 DE 102012112803
(62) Teilanmeldung aus: 13199199.4
(71) Anmelder: Krones AG, 93073 Neutraubling (DE)
(72) Erfinder: Loy, Michael, 93073 Neutraubling (DE)
(74) Vertreter: Bittner, Bernhard

(57) **Zusammenfassung**

Vorrichtung (1) zum Reinigen und/oder Sterilisieren von Kunststoffvorformlingen (10) mit einer Transporteinrichtung (2), welche die Kunststoffvorformlinge (10) entlang eines vorgegebenen Transportpfades (P) transportiert, und mit einer Vielzahl von Beaufschlagungseinrichtungen (4), welche die Kunststoffvorformlinge (10) mit einem fließfähigen Sterilisationsmedium beaufschlagen. Erfindungsgemäß sind die Beaufschlagungseinrichtungen (4) mit den Kunststoffvorformlingen (10) beweglich ausgebildet, wobei die Beaufschlagungseinrichtungen (4) derart angeordnet sind, dass sie das fließfähige Medium unter einem vorgegebenen von 0° verschiedenen Winkel α gegenüber einer Längsrichtung (L) der Kunststoffvorformlinge (10) in diese einspritzen und/oder die Längsrichtungen (LB) der Beaufschlagungseinrichtungen (4) gegenüber den Längsrichtungen (L) der Kunststoffvorformlinge (10) versetzt angeordnet sind.

## Beschreibung

Die vorliegende Erfindung bezieht sich auf eine Vorrichtung und ein Verfahren zum Sterilisieren von Behältnissen und insbesondere zum Sterilisieren bzw. Reinigen von Kunststoffvorformlingen. Im Bereich der Getränke herstellenden Industrie ist es bekannt, dass Kunststoffbehältnisse, insbesondere vor deren Abfüllung, sterilisiert werden. Dabei kann sowohl eine Innenoberfläche der Kunststoffbehältnisse sterilisiert werden als auch eine Außenoberfläche. Die Sterilisierung kann dabei durch keimabtötende Strahlung, wie beispielsweise Beta-Strahlung, erfolgen, aber auch durch Beaufschlagung mit einem Sterilisationsmittel, wie beispielsweise Wasserstoffperoxidgas. Aus dem internen Stand der Technik der Anmelderin ist es mittlerweile jedoch auch bekannt, nicht oder nicht nur die fertig gestellten Kunststoffbehältnisse zu sterilisieren, sondern bereits deren Vorformlinge. Auf diese Weise kann beispielsweise eine gewisse Vorsterilisation dieser Kunststoffvorformlinge erreicht werden, bevor diese zu den Kunststoffbehältnissen expandiert werden.

Um die Haltbarkeit insbesondere von sensiblen Füllprodukten in PET-Behältnissen zu verbessern, muss vor dem Füllprozess die Anzahl der Keime in den Behältern deutlich reduziert werden. Hierfür sind im Stand der Technik verschiedene Nass- und Trockenaseptikmethoden bekannt, die jedoch aufgrund der teilweise großen Flaschenvolumina einen hohen Verbrauch an verwendetem Sterilisationsmedium, wie beispielsweise Peressigsäure oder Wasserstoffperoxid, benötigen. Neuere Maschinenkonzepte reduzieren deshalb bereits die Anzahl der Keime vor dem Blasen der Flasche in dem Kunststoffvorformling. Dabei durchläuft der Kunststoffvorformling einen Behandlungsbereich, in dem die Entkeimung durch gasförmige oder flüssige Sterilisationsmedien oder durch Bestrahlung, wie beispielsweise Bestrahlung mit ultravioletter Strahlung oder Elektronenstrahlung, erzielt wird. Um den Behandlungsbereich klein und kosteneffizient zu gestalten, ist es vorteilhaft, wenn die zu sterilisierenden Behältnisse möglichst schnell komplett mit dem jeweiligen Sterilisationsmittel bedeckt werden. Eine bedeutende Rolle bei dieser Aufgabe spielt insbesondere bei fluidischen Sterilisationsmedien die Gestaltung und Anordnung einer entsprechenden Einspritzdüse, welche das Sterilisationsmedium in die Behältnisse einbringt.

Der vorliegenden Erfindung liegt daher die Aufgabe zugrunde, eine derartige Sterilisation von Kunststoffbehältnissen, insbesondere durch Beaufschlagung mit einem fließfähigen Medium zu verbessern. Diese Aufgaben werden erfindungsgemäß durch die Gegenstände der unabhängigen Ansprüche erreicht.

Vorteilhafte Ausführungsformen und Weiterbildungen sind Gegenstand der Unteransprüche.

Eine erfindungsgemäße Vorrichtung zum Reinigen und/oder Sterilisieren von Kunststoffvorformlingen weist eine Transporteinrichtung auf, welche die Kunststoffvorformlinge entlang eines vorgegebenen Transportpfades transportiert. Weiterhin weist die Vorrichtung eine Vielzahl von Beaufschlagungseinrichtungen auf, welche die Kunststoffvorformlinge mit einem fließfähigen Sterilisationsmedium und insbesondere einem gasförmigen Sterilisationsmedium beaufschlagen.

Erfindungsgemäß ist die Beaufschlagungseinrichtung mit den Kunststoffvorformlingen gemeinsam beweglich ausgebildet, wobei die Beaufschlagungseinrichtungen derart angeordnet sind, dass sie das fließfähige Medium unter einem vorgegebenen von 0° verschiedenen Winkel gegenüber der Längsrichtung der Kunststoffvorformlinge in diese einspritzen und/oder eine bzw. die Längsrichtungen der Beaufschlagungseinrichtungen gegenüber den Längsrichtungen der Kunststoffvorformlinge versetzt angeordnet sind. Es wird daher erfindungsgemäß vorgeschlagen, dass die Beaufschlagungseinrichtungen modifiziert angeordnet sind gegenüber der an sich naheliegenden zentrischen Anordnung und der entsprechenden geradlinigen Einspritzung des Sterilisationsmittels in die Behältnisse.

Wie unten im Detail gezeigt wird, wird durch die erfindungsgemäße Anordnung der Beaufschlagungseinrichtungen eine wirksamere Verteilung des Sterilisationsmediums im Inneren der Kunststoffvorformlinge erreicht. Vorteilhaft sind jedoch die Beaufschlagungseinrichtungen derart angeordnet, dass das aus diesen austretende Sterilisationsmedium im Wesentlichen und bevorzugt vollständig über eine Mündung der Kunststoffvorformlinge in diese eingeführt wird. Auf diese Weise dient die erfindungsgemäße Vorrichtung zum Sterilisieren von Innenoberflächen der Kunststoffvorformlinge. Vorteilhaft ist die Vielzahl der Beaufschlagungseinrichtungen an einem beweglichen Träger angeordnet und besonders bevorzugt werden diese Beaufschlagungseinrichtungen entlang eines geschlossenen Transportpfades transportiert.

Vorteilhaft handelt es sich bei dem beweglichen Träger um einen drehbaren Träger und insbesondere um ein Sterilisationsrad, an dem eine Vielzahl der erwähnten Beaufschlagungseinrichtungen angeordnet ist. Vorteilhaft ist an diesem Träger auch eine Vielzahl von Halteeinrichtungen zum Halten der Kunststoffvorformlinge angeordnet, wobei bevorzugt jeder einzelnen Halteeinrichtung auch eine Beaufschlagungseinrichtung zugeordnet ist. Dies bedeutet, dass gleichwohl während des Sterilisationsvorgangs jede Beaufschlagungseinrichtung gegenüber dem dieser Beaufschlagungseinrichtung zugeordneten Kunststoffvorformling beziehungsweise dessen Halteeinrichtung in einer genau definierten Position angeordnet ist, die sich bevorzugt auch während der Sterilisation der Kunststoffvorformlinge auch nicht ändert.

Generell sind stationäre und mitbewegte Beaufschlagungseinrichtungen bzw. Düsen zu unterscheiden. Bei stationären Beaufschlagungseinrichtungen werden die Kunststoffvorformlinge zur Behandlung unter den Düsen vorbei transportiert. Auf diese Weise ergibt sich eine relativ geringe zur Verfügung stehende Einspritzzeit. Bei einer kontinuierlichen Düsenausströmung trifft zudem ein Großteil des Sterilisationsmediums auf die Zwischenräume, wodurch hohe Verluste entstehen. Bei einer getakteten Einspritzung ist eine aufwendige mechanische Lösung erforderlich. Durch das Mitführen der Beaufschlagungseinrichtungen kann umgekehrt die Zeit für die Beaufschlagung erhöht werden und dies auch bei hohen Transportgeschwindigkeiten der Kunststoffvorformlinge. Bevorzugt sind die Beaufschlagungseinrichtungen über den jeweiligen Halteeinrichtungen bzw. Kunststoffvorformlingen angeordnet. Während des Transports, also während der Rotation des drehbaren Trägers, sind die Beaufschlagungseinrichtungen bevorzugt immer oberhalb des jeweiligen Kunststoffvorformlings mitlaufend geführt.

Mit anderen Worten ermöglichen mitbewegte Beaufschlagungseinrichtungen bzw. Düsen bei kontinuierlichem Ausströmen längere Behandlungszeiten. Durch die Bewegung der Beaufschlagungseinrichtung und des Kunststoffvorformlings bildet sich jedoch in einem dazwischen bestehenden Spalt eine Strömung aus, die, wie unten genauer erläutert wird, bei der Verwendung von insbesondere gasförmigen Sterilisationsmedien einen störenden Einfluss auf die optimale Durchströmung der Kunststoffvorformlinge haben kann. Die vorliegende Erfindung sieht insbesondere eine Sterilisation der Kunststoffvorformlinge durch ein gasförmiges Sterilisationsmedium vor. Dabei wird sowohl der Kunststoffvorformling als auch die Beaufschlagungseinrichtung in der Transportrichtung bewegt. Um den störenden Einfluss der entstehenden Spaltströmung zu minimieren, ist die Beaufschlagungseinrichtung um den Winkel α gegen die Transportrichtung angestellt und/oder vorteilhaft gegenüber der Mitte des Kunststoffvorformlings exzentrisch versetzt und insbesondere exzentrisch entlang der Transportrichtung versetzt.

Bei einer bevorzugten Ausführungsform sind die Beaufschlagungseinrichtungen derart angeordnet, dass sie das fließfähige Medium unter einem vorgegebenen von 0° verschiedenen Winkel gegenüber der Längsrichtung der Kunststoffvorformlinge in diese einspritzen und eine Längsrichtung der Beaufschlagungseinrichtungen gegenüber den Längsrichtungen der Kunststoffvorformlinge versetzt angeordnet ist. Es wird damit hier eine Kombination der oben genannten Maßnahmen, d.h. der Schrägstellung der Beaufschlagungseinrichtung und auch deren Versatz gegenüber der Längsrichtung der Kunststoffvorformlinge vorgeschlagen. Die Anmelderin konnte herausfinden, dass gerade auch durch eine Kombination dieser beiden Maßnahmen ein sehr effizientes Einspritzen des Sterilisationsmediums in die Kunststoffvorformlinge erreicht wird.

Bei einer weiteren vorteilhaften Ausführungsform sind die Beaufschlagungseinrichtungen derart angeordnet, dass sie das fließfähige Sterilisations- bzw. Reinigungsmedium unter einem vorgegebenen von 0° verschiedenen Winkel gegenüber der Längsrichtung der Kunststoffvorformlinge einspritzen, bevorzugt derart, dass die Einspritzrichtung auch eine Komponente aufweist, die entgegen der Transportrichtung der Kunststoffvorformlinge gerichtet ist.

Bei dieser Vorgehensweise wird vorgeschlagen, dass auch mit einer Komponente entgegen der Bewegungsrichtung der Kunststoffvorformlinge eingespritzt wird. Dabei ist es möglich, dass die Schrägstellung in einer Ebene ausgebildet ist, welche auch eine Bewegungsrichtung der Kunststoffvorformlinge sowie auch eine Längsrichtung der zu sterilisierenden Kunststoffvorformlinge enthält.

Bei einer weiteren vorteilhaften Ausführungsform ist eine Längsrichtung der Beaufschlagungseinrichtungen in der Bewegungsrichtung der Kunststoffvorformlinge jeweils hinter den Längsrichtungen der Kunststoffvorformlinge angeordnet. Bei dieser Ausgestaltung wird vorgeschlagen, dass die einzelnen Beaufschlagungseinrichtungen gegenüber den ihnen zugeordneten Kunststoffvorformlingen bzw. gegenüber den von diesen zu sterilisierenden Kunststoffvorformlingen jeweils betrachtet in der Transporteinrichtung der Kunststoffvorformlinge nach hinten versetzt sind. Damit eilen die Beaufschlagungseinrichtungen den ihnen jeweils zugeordneten Kunststoffvorformlingen in der Bewegungsrichtung der Kunststoffvorformlinge jeweils (zumindest geringfügig) nach.

Bei einer weiteren vorteilhaften Ausführungsform ist zwischen den Mündungen der Kunststoffvorformlinge und den Beaufschlagungseinrichtungen ein sich in der Transporteinrichtung der Kunststoffvorformlinge erstreckender Spalt ausgebildet. Genauer gesagt kann dieser Spalt auch zwischen einem Träger der Beaufschlagungseinrichtungen und den Kunststoffvorformlingen ausgebildet sein. Dieser Spalt dient dabei ebenfalls zum Erzeugen der Strömung, die durch die Kunststoffvorformlinge zu der Sterilisation geführt wird. Es wäre dabei möglich, dass die Beaufschlagungseinrichtungen zumindest auch geringfügig in die Mündungen der Kunststoffvorformlinge hineinragen. Vorteilhaft sind die Beaufschlagungseinrichtungen bzw. die Düsen jedoch oberhalb der Mündungsränder der Kunststoffvorformlinge bzw. von diesen Mündungsrändern beabstandet angeordnet.

Vorteilhaft liegt die Höhe des besagten Spalts zwischen 0,5 mm und 10 mm, bevorzugt zwischen 1 mm und 8 mm, bevorzugt zwischen 1 mm und 5 mm und besonders bevorzugt zwischen 1 mm und 3 mm.

Bei einer weiteren bevorzugten Ausführungsform sind die Beaufschlagungseinrichtungen an einem Träger angeordnet. Dieser Träger ist dabei beweglich und bewegt bevorzugt ebenfalls die Kunststoffvorformlinge. Wie oben erwähnt, handelt es sich hierbei bevorzugt um einen kreisförmigen Träger, der bezüglich einer Drehachse gedreht wird.

Vorteilhaft ist der vorgegebene Winkel, unter dem das Sterilisationsmittel eingeströmt wird, zwischen 5° und 50°, bevorzugt 10° und 30°, bevorzugt zwischen 10° und 20° und besonders bevorzugt zwischen 12° und 18° bezüglich der Längsrichtung der Kunststoffvorformlinge. Durch diesen Winkel kann erreicht werden, dass das insbesondere gasförmige Sterilisationsmedium in vergleichsweise sehr kurzer Zeit bis zu dem Boden der Kunststoffvorformlinge in diese gelangt und auch durch eine entsprechend geführte Strömung wieder aus den Kunststoffvorformlingen heraus gelangt. Auf diese Weise ist eine weitgehende Benetzung bzw. Beaufschlagung der Innenwandung der Kunststoffvorformlinge mit dem Sterilisationsmittel möglich.

Bei einer weiteren bevorzugten Ausführungsform ist ein Abstand d zwischen der Längsrichtung der Kunststoffvorformlinge und der Längsrichtung der Beaufschlagungseinrichtungen zwischen 2 mm und 12 mm, bevorzugt zwischen 4 mm und 10 mm, besonders bevorzugt zwischen 5 mm und 9 mm, wobei jedoch auch dieser Abstand insbesondere auch von dem Mündungsquerschnitt der zu sterilisierenden Kunststoffvorformlinge abhängt. Vorteilhaft werden die Kunststoffvorformlinge in vertikaler Ausrichtung transportiert und besonders bevorzugt sind die Beaufschlagungseinrichtungen jeweils oberhalb der ihnen jeweils zugeordneten Kunststoffvorformlinge angeordnet.

Bei einer weiteren vorteilhaften Ausführungsform ist der vorgegebene Winkel und/oder der Versatz zwischen der Längsrichtung der Kunststoffvorformlinge und den Längsrichtungen der Beaufschlagungseinrichtungen einstellbar. Bevorzugt sind dabei diese Parameter auch veränderbar. Bei einer weiteren bevorzugten Ausführungsform ist auch die besagte Höhe des Spalts oberhalb der Mündungen der Kunststoffvorformlinge veränderbar. Bevorzugt ist daher auch die Spalthöhe einstellbar. Auf diese Weise kann eine Einstellung auf unterschiedliche Kunststoffvorformlingsgeometrien erreicht werden. Vorteilhaft ist dabei eine Verstelleinrichtung vorgesehen, welche eine gemeinsame oder getrennte Veränderung der besagten Parameter der einzelnen Beaufschlagungseinrichtungen ggfs. auch automatisch ermöglicht.

So wäre es beispielsweise möglich, dass die Beaufschlagungseinrichtungen auf einem gemeinsamen Träger angeordnet sind, der wiederum drehbeweglich gegenüber einem weiteren Träger, an dem die Kunststoffvorformlinge angeordnet sind, angeordnet ist, so dass auf diese Weise bereits der Versatz der Beaufschlagungseinrichtungen einheitlich geändert werden kann. Weiterhin wäre es möglich, dass die Beaufschlagungseinrichtungen an zwei bezüglich einander drehbaren Trägern angeordnet sind, wobei durch eine Drehbarkeit dieser beiden Träger gegeneinander auch eine gemeinsame Verstellung der Winkel bzw. Einspritzeinrichtungen sämtlicher Beaufschlagungseinrichtungen erreicht wird.

Bei einer weiteren vorteilhaften Ausführungsform weisen die Beaufschlagungseinrichtungen einen Bohrungsdurchmesser bzw. Austrittsdurchmesser für das Sterilisationsmedium auf, der zwischen 3 mm und 8 mm liegt.

Allgemein weist bevorzugt die Vorrichtung eine Umlenkungseinrichtung auf, welche wieder aus dem Kunststoffbehältnis austretendes Sterilisationsmedium auf einen Außenbereich der Mündung des Kunststoffbehältnisses lenkt.

Bei einer weiteren vorteilhaften Ausführungsform ist angrenzend zu der Mündung der Kunststoffbehältnisse ein Träger angeordnet, wobei dieser Träger an einer der Mündungen der Kunststoffbehältnisse eine Ausnehmung aufweist, welche sich in einer radialen Richtung des Kunststoffbehältnisses über dessen Mündungsrand hinweg erstreckt. Das aus dem Kunststoffbehältnis wieder austretende Sterilisationsmedium kann durch diese Ausnehmung auch auf eine Außenfläche des Gewindes des Kunststoffbehältnisses gelenkt werden und so auch diese sterilisieren. Vorteilhaft weist diese Ausnehmung ein gekrümmtes Profil auf, welches eine entsprechende Umlenkung des Sterilisationsmediums bewirkt.

Es wird darauf hingewiesen, dass diese Ausgestaltung mit der Ausnehmung oder allgemein mit der Umlenkungseinrichtung auch unabhängig von der oben beschriebenen Ausgestaltung der Beaufschlagungseinrichtung bzw. Düse Anwendung finden kann. Die Anmelderin behält sich daher vor, auch auf eine derartige Ausführungsform Schutz zu beanspruchen.

Vorteilhaft ist der besagte Träger oberhalb der Kunststoffbehältnisse angeordnet. Bei einer weiteren vorteilhaften Ausführungsform weist der Träger auch einen auf die Kunststoffbehältnisse bzw. Kunststoffvorformlinge zuweisenden Vorsprung auf.

Dabei ist es möglich, dass eine derartige Ausnehmung vorgesehen ist, welche sich oberhalb der Mündungen aller Kunststoffbehältnisse erstreckt. Dabei ist es möglich, dass eine erste derartige Ausnehmung bzw. Nut vorgesehen ist, welche bezüglich der Längsrichtungen der Kunststoffbehältnisse radial innerhalb deren Längsrichtungen angeordnet ist und bevorzugt auch eine zweite Nut vorgesehen ist, welche bezüglich der Längsrichtungen der Kunststoffvorformlinge außerhalb deren Längsrichtungen angeordnet ist.

Es wäre jedoch auch möglich, dass jeder einzelnen Beaufschlagungseinrichtung jeweils eine ringförmige Nut bzw. Ausnehmung zugeordnet ist, welche sich ringförmig um die Längsrichtung des jeweiligen Kunststoffbehältnisses erstreckt.

Die vorliegende Erfindung ist weiterhin auf ein Verfahren zum Reinigen und/oder Sterilisieren von Kunststoffvorformlingen gerichtet. Dabei werden die Kunststoffvorformlinge entlang eines vorgegebenen Pfades transportiert und während dieses Transports wird ein Innenraum dieser Kunststoffvorformlinge von einer Vielzahl von Beaufschlagungseinrichtungen mit einem fließfähigen und insbesondere gasförmigen Sterilisationsmittel beaufschlagt. Erfindungsgemäß wird das fließfähige Medium unter einem vorgegebenen von 0° verschiedenen Winkel gegenüber der Längsrichtung der Kunststoffvorformlinge in diese eingegeben und/oder die Längsrichtungen der Beaufschlagungseinrichtungen werden gegenüber den Längsrichtungen der Kunststoffvorformlinge während der Beaufschlagung versetzt angeordnet.

Es wird daher auch verfahrensseitig vorgeschlagen, dass die Beaufschlagung der Innenräume der Kunststoffvorformlinge nicht in der an sich naheliegenden zentrischen und in Längsrichtung der Kunststoffvorformlinge erfolgenden Weise durchgeführt wird, sondern hier gegenüber modifiziert wird. Vorteilhaft wird das fließfähige Sterilisationsmedium unter dem vorgegebenen Winkel gegenüber der Längsrichtung eingespritzt. Zudem sind die Beaufschlagungseinrichtungen gegenüber den Längsrichtungen der Kunststoffvorformlinge versetzt angeordnet.

Vorteilhaft ist zumindest derjenige Abschnitt der Beaufschlagungseinrichtung, aus dem das Sterilisationsmedium austritt, in der oben bezeichneten Weise gegenüber der Längsrichtung der Kunststoffvorformlinge winklig angeordnet, dass die Austrittsrichtung, die durch diesen Abschnitt definiert wird, den oben erwähnten Winkel gegenüber der Längsrichtung der Kunststoffvorformlinge aufweist. Vorteilhaft bewegen sich die Beaufschlagungseinrichtungen gemeinsam mit den Kunststoffvorformlingen.

Vorteilhaft wird durch das hier beschriebene Verfahren eine Vorsterilisierung der Kunststoffbehältnisse bzw. Kunststoffvorformlinge vorgenommen. Vorteilhaft werden die Kunststoffvorformlinge im Anschluss an die hier beschriebene Sterilisation mittels einer Vorrichtung zum Umformen von Kunststoffvorformlingen zu Kunststoffbehältnissen zu diesen Kunststoffbehältnissen umgeformt und bevorzugt werden die erzeugten Kunststoffbehältnisse im Anschluss hieran nochmals sterilisiert. Auch diese weitere Sterilisierung kann mittels eines fließfähigen Sterilisationsmediums erfolgen.

Wie oben erwähnt, weist bevorzugt die Einspritzrichtung des Sterilisationsmediums auch eine Komponente in einer der Transportrichtung der Kunststoffvorformlinge entgegen gesetzten Richtung auf.

Vorteilhaft wird zwischen den Mündungsrändern der Kunststoffvorformlinge und einem Träger, an dem die Beaufschlagungseinrichtungen angeordnet sind, eine Strömung durch die Bewegung der Kunststoffvorformlinge erzeugt, welche ebenfalls für eine solche Strömung in den Kunststoffvorformlingen sorgt, welche bewirkt, dass zumindest Großteile der Innenwandung der Kunststoffvorformlinge mit dem gasförmigen Sterilisationsmedium beaufschlagt werden. Es wäre daneben auch möglich, dass die Kunststoffvorformlinge während der Sterilisation um ihre eigene Längsrichtung gedreht werden.

Vorteilhaft werden die Kunststoffvorformlinge während ihrer Sterilisation oberhalb des Tragrings im Bereich des Mundstücks gehalten.. Bei dem Tragring handelt es sich insbesondere um einen unterhalb des Gewindes des Kunststoffbehältnisses ausgebildeten umlaufenden Ring, der in radialer Richtung hervorsteht. Dieser Ring dient insbesondere zum Transportieren und zur Übergabe der Kunststoffbehältnisse bzw. -vorformlinge. Bei einem weiteren vorteilhaften Verfahren werden die Kunststoffvorformlinge wenigstens teilweise entlang eines kreisförmigen Transportpfades bewegt.

Weitere Vorteile und Ausführungsformen ergeben sich aus den beigefügten Zeichnungen.

Darin zeigen:
- Fig. 1: Eine schematische Darstellung einer erfindungsgemäßen Vorrichtung zum Sterilisieren von Kunststoffvorformlingen;
- Fig. 2: eine grob schematische Darstellung zur Veranschaulichung der Einspritzrichtung der Beaufschlagungseinrichtung;
- Fig. 3: zwei Darstellungen zur Gegenüberstellung und zur Veranschaulichung der durch die erfindungsgemäße Einspritzung erreichten Ergebnisse;
- Fig. 4: eine Darstellung einer erfindungsgemäßen Vorrichtung mit einer Umlenkungseinrichtung zum Umlenken der aus dem Behältnis austretenden Strömung auf den Außenbereich des Kunststoffbehältnisses; und;
- Fig. 5: eine Darstellung der in Fig. 4 gezeigten Umlenkeinrichtung.

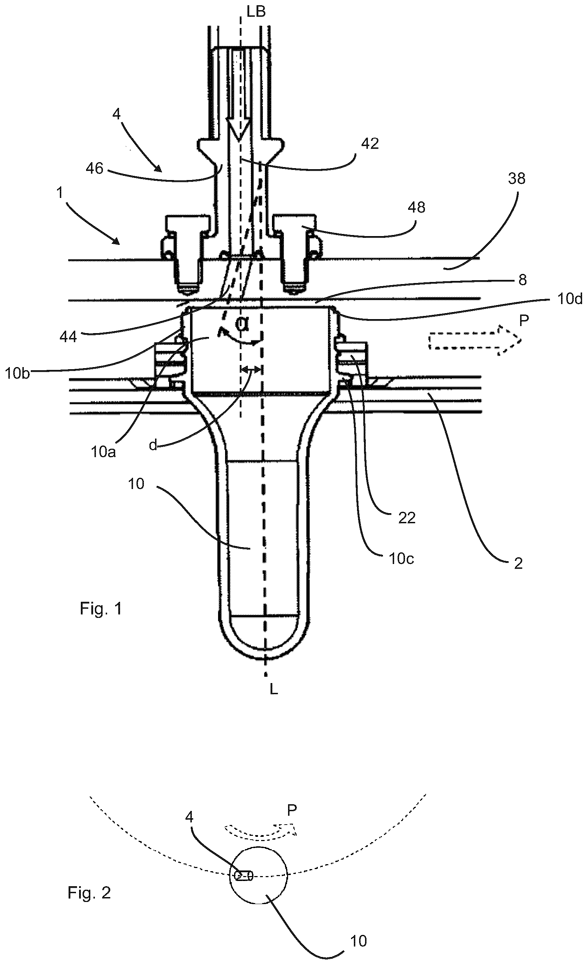

Fig. 1 zeigt eine teilweise Darstellung einer erfindungsgemäßen Vorrichtung 1 zum Sterilisieren von Kunststoffvorformlingen 10. Dabei bezieht sich das Bezugszeichen L auf eine Längsrichtung der Kunststoffvorformlinge, die hier gleichzeitig auch eine Symmetrieachse der Kunststoffvorformlinge darstellt. Diese Kunststoffvorformlinge 10 weisen einen Mündungsabschnitt 10a auf, der gegenüber einem Grundkörper der Kunststoffvorformlinge einen vergrößerten Querschnitt aufweist. Unterhalb eines Gewindes 10b der Kunststoffvorformlinge ist ein Tragring 10c vorgesehen. Das Bezugszeichen 22 kennzeichnet eine Halteeinrichtung, welche den Kunststoffvorformling in einem Bereich seiner Mündung, beispielsweise einem Bereich unterhalb seines Tragrings, hält. Das Bezugszeichen 2 kennzeichnet einen Träger, an dem der Kunststoffvorformling 10 während seiner Sterilisation angeordnet ist. Bei diesem Träger 2 kann es sich bevorzugt, wie oben erwähnt, um ein Trägerrad handeln. Das Bezugszeichen P kennzeichnet die Transportrichtung der Kunststoffvorformlinge.

Oberhalb der Mündungen der Kunststoffvorformlinge 10 ist eine in ihrer Gesamtheit mit 4 bezeichnete Beaufschlagungseinrichtung vorgesehen, welche die Innenräume der Kunststoffvorformlinge 10 mit einem Sterilisationsmittel beaufschlagt. Mittels Befestigungseinrichtungen 48, beispielsweise Schrauben, kann dabei diese Beaufschlagungseinrichtung 4 an einem Träger 38 befestigt sein. Dieser Träger 38 kann sich wiederum gemeinsam mit der Transporteinrichtung 2 bewegen, so dass die Beaufschlagungseinrichtung 4 stets in der gezeigten Position oberhalb des Kunststoffvorformlings 10 angeordnet ist. Es wird jedoch darauf hingewiesen, dass entsprechend auch eine Beaufschlagung in anderen Orientierungen der Kunststoffvorformlinge 10, beispielsweise, wenn diese umgedreht sind, möglich wäre.

Das Bezugszeichen LB kennzeichnet eine Längsrichtung der Beaufschlagungseinrichtung sowie auch eine Längsrichtung eines Kanals 42, über welchen das Sterilisationsmittel zugeführt wird. Dieser Kanal 42 ist im Inneren eines Grundkörpers 46 der Beaufschlagungseinrichtung bzw. Düseneinrichtung 4 ausgebildet. Das Bezugszeichen 44 kennzeichnet eine Düseneinrichtung, über welche das Sterilisationsmittel in das Innere der Kunststoffvorformlinge gelangt. Man erkennt, dass diese Düseneinrichtung 44 in einem Winkel α gegenüber der Längsrichtung L der Kunststoffvorformlinge 10 angeordnet ist. Die Längsrichtung L ist hier auch eine vertikale Richtung.

Das Bezugszeichen 8 kennzeichnet einen Spalt, der zwischen dem Träger 38 und insbesondere zwischen einer Austrittsöffnung der Beaufschlagungseinrichtung bzw. Düseneinrichtung 44 und dem oberen Mündungsrand der Kunststoffvorformlinge ausgebildet ist. Es wäre jedoch auch möglich, dass die Düseneinrichtung 44 nach unten über den Träger 38 hinaus steht, da die Kunststoffvorformlinge 10 gemeinsam mit den Beaufschlagungseinrichtungen transportiert werden Der Spalt 8 erstreckt sich damit in einer horizontalen Ebene.

Man erkennt damit, dass die Einspritzrichtung des Sterilisationsmediums einerseits in dem Winkel α gegenüber der Längsrichtung L, d.h. insbesondere auch schräg gegenüber der Transportrichtung, des Kunststoffvorformlings 10 angeordnet ist und dass andererseits die Längsrichtung LB der Beaufschlagungseinrichtung 4 gegenüber der Längsrichtung L des Kunststoffvorformlings 10 nach hinten, das heißt entgegen der Transportrichtung P versetzt ist. Der Versatz ergibt sich hier durch den Abstand d zwischen den (zueinander parallelen) Längsrichtungen L und LB.

Fig. 2 zeigt eine schematische Darstellung zur Veranschaulichung der Anordnung der Beaufschlagungseinrichtung. Man erkennt auch an dieser Darstellung, dass die Beaufschlagungseinrichtung 4 einerseits gegenüber dem Zentrum des Kunststoffvorformlings 10 und damit auch gegenüber dessen Längsrichtung entgegen der Transportrichtung P versetzt ist. Auch erkennt man in Fig. 2 die schräge Anstellung der Beaufschlagungseinrichtung 4 bzw. des Düsenabschnitts 44 bzw. die sich dadurch ergebende schräge Beaufschlagungsrichtung des Sterilisationsmediums. Auch erkennt man, dass die Kunststoffvorformlinge 10 entlang eines kreisförmigen Transportpfades transportiert werden.

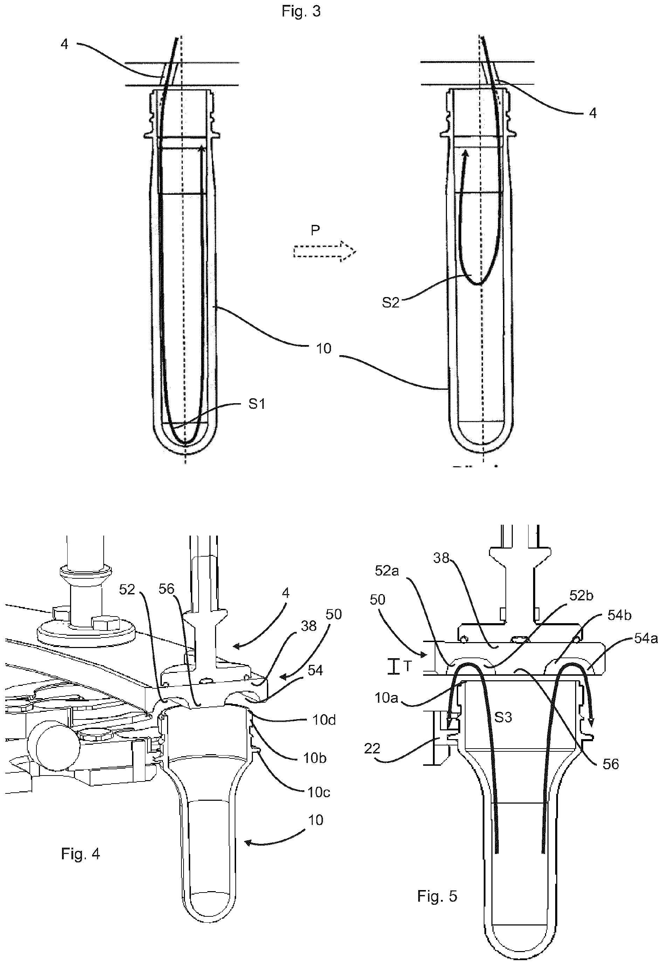

Fig. 3 zeigt eine Gegenüberstellung zur Veranschaulichung der durch die Erfindung erzielten Wirkung. Wie bereits oben erwähnt, wird während der Sterilisation sowohl der Kunststoffvorformling 10 als auch die Düse in der Transportrichtung bewegt. Um den störenden Einfluss der entstehenden Spaltströmung zu minimieren, ist dabei, wie oben erwähnt, die Düse um den Winkel α gegen die Transportrichtung angestellt und auch gegenüber der Mitte exzentrisch entlang der Transportrichtung versetzt.

Um das Prozessgas bzw. das Sterilisationsmittel möglichst schnell auf der gesamten Innenoberfläche des Kunststoffvorformlings zu verteilen, wird die Strömung auf kurzem Weg an die Innenwandung des Kunststoffvorformlings treffen. Dadurch entsteht in der Grenzschicht eine laminare Strömung, die an der Innenwand des Kunststoffvorformlings in kurzer Zeit bis an den Boden gelangt. Dies ist in der linken Darstellung von Fig. 3 veranschaulicht. Das Bezugszeichen S1 kennzeichnet den Strömungsverlauf des Sterilisationsmediums bei der erfindungsgemäßen Anordnung der Beaufschlagungseinrichtung.

Bei einer schrägen bzw. schiefen Anstellung der Düse 44 gegen die Transportrichtung unterstützt die entstehende Spaltströmung die Anlagerung des Prozessgasstromes. Bei einer Anstellung in Transportrichtung wirkt die Spaltströmung dem Anlagern entgegen, wie durch die Darstellung in dem rechten Bild von Fig. 3 gezeigt ist (Strömungsverlauf S2). In diesem Falle wird die Prozessgasströmung zunehmend turbulent und wird damit weniger Anteile des Sterilisationsmediums an den Boden der Kunststoffvorformlinge bringen. Dies ist ebenfalls durch die rechte Darstellung in Fig. 3 gezeigt. Zur Optimierung des Systems ist es weiterhin denkbar, dass die Exzentrizität zur Anpassung an verschiedene Mündungsstückdurchmesser und/oder Stationsleistungen einstellbar gestaltet wird.

Fig. 4 zeigt eine weitere Vorrichtung zum Sterilisieren von Behältnissen, wobei hier auch eine Umlenkeinrichtung 50 vorgesehen ist, welche das aus den Behältnissen austretende Sterilisationsmedium auf eine Außenoberfläche des Behältnisses 10, insbesondere einen Gewindebereich 10b des Behältnisses umlenkt.

Zu diesem Zweck sind hier in dem Träger 38 an der den Kunststoffbehältnissen 10 zugewandten Seite 38a zwei Nuten bzw. Ausnehmungen 52, 54 angeordnet. Das aus dem Behältnis austretende gasförmige Medium wird durch diese Nuten abgelenkt und gelangt so auf das Gewinde 10b der Kunststoffbehältnisse.

Man erkennt, dass sich die beiden Nuten 52, 54 über den Mündungsrand 10d des Kunststoffbehältnisses hinweg erstrecken.

Fig. 5 zeigt eine Schnittdarstellung dieser Umlenkeinrichtung 50. Man erkennt, dass der jeweils radial größere Bereich 52b, 54b der beiden Nuten 52, 54 radial innerhalb des Behältnisses bzw. dessen Mündungsquerschnitts liegt und der jeweils radial kleinere Bereich 52a, 54a radial außerhalb. Auf diese Weise kann ein relativ großer Anteil des Sterilisationsmediums "eingefangen" und umgelenkt werden. Durch den relativ geringen radialen Anteil 52b, 54b kann eine entsprechend gezielte Umlenkung erreicht werden. Im Querschnitt weisen die beiden Ausnehmung bevorzugt die Gestalt einer Ellipsenhälfte auf. Durch die oben beschriebene Ausgestaltung der Nuten 52, 54 ist es möglich, eine Vielzahl von Kunststoffvorformlingen mit verschiedenen Mündungsdurchmessern mit der Umlenkeinrichtung 50 behandeln zu können.

Das Bezugszeichen 10d kennzeichnet einen Mündungsrand der Kunststoffbehältnisse. Man erkennt, dass sich die beiden Nuten 52, 54 jeweils in einer radialen Richtung des Kunststoffbehältnisses über diesen Mündungsrand 10d hinweg erstrecken.

Damit kann durch die beiden radial versetzten Nuten 52, 54 die Entkeimung der Außenoberfläche des Kunststoffbehältnisses verbessert werden. Bei der hier gezeigten Ausführungsform erstrecken sich die beiden Nuten 52, 54 ringförmig entlang des Trägers 38. Zwischen den beiden Nuten 52, 54 ist ein Vorsprung 56 vorgesehen, der in Richtung der Mündung des Kunststoffbehältnisses (hier nach unten) hervorsteht. In diesem Vorsprung befinden sich die jeweiligen Düsen, durch welche den Kunststoffvorformlingen das Sterilisationsmedium zugeführt wird.

Bevorzugt liegt eine maximale Tiefe T dieser Ausnehmungen zwischen 2 und 10 mm. Das Bezugszeichen S3 kennzeichnet den Strömungsverlauf des aus dem Kunststoffbehältnis bzw. dem Kunststoffvorformlinge wieder herausgelangenden Sterilisationsmediums.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Vorrichtung
- 2: Träger/Transporteinrichtung
- 4: Beaufschlagungseinrichtung
- 8: Spalt
- 10: Kunststoffvorformlinge
- 10a: Mündungsabschnitt
- 10b: Gewinde
- 10c: Tragring
- 10d: Mündungsrand
- 22: Halteeinrichtung
- 38: Träger
- 42: Kanal
- 44: Düseneinrichtung
- 46: Grundkörper
- 48: Befestigungseinrichtung
- 50: Umlenkungseinrichtung
- 52: erste Nut in dem Träger 38
- 52a, 52b: Bereiche der ersten Nut
- 54: zweite Nut in dem Träger
- 54a, 54b: Bereiche der zweiten Nut
- 56: Vorsprung zwischen den Nuten 52, 54
- P: Transportrichtung
- LB: Längsrichtung der Beaufschlagungseinrichtung
- L: Längsrichtung des Kunststoffvorformlings
- d: Abstand zwischen den Längsrichtungen L und LB
- α: Winkel
- S1, S2, S3: Strömungsverläufe
- T: Tiefe der Nut

## Patentansprüche

1. Vorrichtung (1) zum Reinigen und/oder Sterilisieren von Kunststoffvorformlingen (10) mit einer Transporteinrichtung (2), welche die Kunststoffvorformlinge (10) entlang eines vorgegebenen Transportpfades (P) transportiert, und mit einer Vielzahl von Beaufschlagungseinrichtungen (4), welche die Kunststoffvorformlinge (10) mit einem fließfähigen Sterilisationsmedium beaufschlagen,
**dadurch gekennzeichnet, dass**
die Beaufschlagungseinrichtungen (4) mit den Kunststoffvorformlingen (10) beweglich ausgebildet sind, wobei die Beaufschlagungseinrichtungen (4) derart angeordnet sind, dass sie das fließfähige Medium unter einem vorgegebenen von 0° verschiedenen Winkel α gegenüber einer Längsrichtung (L) der Kunststoffvorformlinge (10) in diese einspritzen und/oder die Längsrichtungen (LB) der Beaufschlagungseinrichtungen (4) gegenüber den Längsrichtungen (L) der Kunststoffvorformlinge (10) versetzt angeordnet sind, wobei zwischen den Mündungen der Kunststoffvorformlinge (10) und den Beaufschlagungseinrichtungen (4) ein sich in der Transportrichtung (P) der Kunststoffvorformlinge (10) erstreckender Spalt (8) ausgebildet ist, und weiter wobei die Höhe des Spalts (8) zwischen den Kunststoffvorformlingen (10) und den Beaufschlagungseinrichtungen (4) einstellbar ist.

2. Vorrichtung (1) nach Anspruch 1,
**dadurch gekennzeichnet, dass**
der vorgegebene Winkel und/oder der Versatz zwischen der Längsrichtung der Kunststoffvorformlinge und den Längsrichtungen der Beaufschlagungseinrichtungen und/oder die Höhe des Spalts zwischen den Kunststoffvorformlingen und der Beaufschlagungseinrichtung einstellbar ist/sind, insbesondere wobei dabei diese Parameter auch veränderbar sind, insbesondere weiter wobei die besagte Höhe des Spalts oberhalb der Mündungen der Kunststoffvorformlinge veränderbar ist, wobei weiter bevorzugt daher auch die Spalthöhe einstellbar ist, sodass auf diese Weise eine Einstellung auf unterschiedliche Kunststoffvorformlingsgeometrien erreicht werden kann, wobei vorteilhaft dabei eine Verstelleinrichtung vorgesehen ist, welche eine gemeinsame oder getrennte Veränderung der besagten Parameter der einzelnen Beaufschlagungseinrichtungen ggfs. auch automatisch ermöglicht.

3. Vorrichtung (1) nach wenigstens einem der vorangegangenen Ansprüche,
**dadurch gekennzeichnet, dass**
die Beaufschlagungseinrichtungen (4) derart angeordnet sind, dass das aus diesen austretende Sterilisationsmedium im Wesentlichen und bevorzugt vollständig über eine Mündung der Kunststoffvorformlinge (10) in diese eingeführt wird, sodass auf diese Weise die erfindungsgemäße Vorrichtung (1) zum Sterilisieren von Innenoberflächen der Kunststoffvorformlinge (10) dient.

4. Vorrichtung (1) nach wenigstens einem der vorangegangenen Ansprüche,
**dadurch gekennzeichnet, dass**
eine Längsrichtung (LB) der Beaufschlagungseinrichtungen (4) in der Bewegungsrichtung der Kunststoffvorformlinge (10) gegenüber den Längsrichtungen der Kunststoffvorformlinge (L) bezüglich der Transportrichtung der Kunststoffvorformlinge nach hinten versetzt angeordnet ist.

5. Vorrichtung (1) nach wenigstens einem der vorangegangenen Ansprüche,
**dadurch gekennzeichnet, dass**
die Beaufschlagungseinrichtungen (4) an einem, insbesondere beweglichen, Träger (38) angeordnet sind.

6. Vorrichtung (1) nach wenigstens einem der vorangegangenen Ansprüche,
**dadurch gekennzeichnet, dass**
es sich bei dem beweglichen Träger (38) um einen drehbaren Träger und insbesondere um ein Sterilisationsrad handelt, an dem eine Vielzahl der erwähnten Beaufschlagungseinrichtungen (4) angeordnet sind, insbesondere wobei an diesem Träger (38) auch eine Vielzahl von Halteeinrichtungen (22) zum Halten der Kunststoffvorformlinge (10) angeordnet sind, wobei bevorzugt jeder einzelnen Halteeinrichtung (22) auch eine Beaufschlagungseinrichtung (4) zugeordnet ist, was bedeutet, dass gleichwohl während des Sterilisationsvorgangs jede Beaufschlagungseinrichtung (4) gegenüber dem dieser Beaufschlagungseinrichtung (4) zugeordneten Kunststoffvorformling (10) beziehungsweise dessen Halteeinrichtung (22) in einer genau definierten Position angeordnet ist, die sich bevorzugt auch während der Sterilisation der Kunststoffvorformlinge (10) auch nicht ändert.

7. Vorrichtung (1) nach wenigstens einem der vorangegangenen Ansprüche,
**dadurch gekennzeichnet, dass**
die Beaufschlagungseinrichtungen (4) über den jeweiligen Halteeinrichtungen (22) bzw. Kunststoffvorformlingen (10) angeordnet sind, wobei während des Transports, also während der Rotation des, insbesondere drehbaren, Trägers (38), die Beaufschlagungseinrichtungen (4) bevorzugt immer oberhalb des jeweiligen Kunststoffvorformlings (10) mitlaufend geführt werden.

8. Vorrichtung (1) nach wenigstens einem der vorangegangenen Ansprüche,
**dadurch gekennzeichnet, dass**,
um den störenden Einfluss der entstehenden Spaltströmung zu minimieren, die Beaufschlagungseinrichtungen (4) um den Winkel α gegen die Transportrichtung (P) angestellt und/oder vorteilhaft gegenüber der Mitte des Kunststoffvorformlings (10) exzentrisch versetzt und insbesondere exzentrisch entlang der Transportrichtung (P) versetzt sind.

9. Vorrichtung (1) nach wenigstens einem der vorangegangenen Ansprüche,
**dadurch gekennzeichnet, dass**
der vorgegebene Winkel α zwischen 5° und 50°, bevorzugt zwischen 10° und 30°, bevorzugt zwischen 10° und 20° und besonders bevorzugt zwischen 12° und 18° liegt.

10. Vorrichtung (1) nach wenigstens einem der vorangegangenen Ansprüche,
**dadurch gekennzeichnet, dass**
der Abstand zwischen der Längsrichtung (L) der Kunststoffvorformlinge (10) und der Längsrichtung (LB) der Beaufschlagungseinrichtungen (4) zwischen 2mm und 12mm, bevorzugt zwischen 4mm und 10mm und besonders bevorzugt zwischen 5mm und 9mm liegt.

11. Vorrichtung (1) nach wenigstens einem der vorangegangenen Ansprüche,
**dadurch gekennzeichnet, dass**
die Höhe des besagten Spalts zwischen 0,5mm und 10mm, bevorzugt zwischen 1mm und 8mm, bevorzugt zwischen 1mm und 5mm und besonders bevorzugt zwischen 1mm und 3mm liegt.

12. Vorrichtung (1) nach wenigstens einem der vorangegangenen Ansprüche,
**dadurch gekennzeichnet, dass**
die Vorrichtung (1) eine Umlenkeinrichtung (50) aufweist, welche aus dem Kunststoffbehältnis (10) austretendes Sterilisationsmedium auf einen Außenbereich des Kunststoffbehältnisses (10) umlenkt.

13. Vorrichtung (1) nach wenigstens einem der vorangegangenen Ansprüche,
**dadurch gekennzeichnet, dass**
angrenzend zu der Mündung der Kunststoffbehältnisse (10) ein Träger (38) angeordnet ist, wobei dieser Träger an einer der Mündungen der Kunststoffbehältnisse (10) wenigstens eine Ausnehmung (52, 54) aufweist, welche sich in einer radialen Richtung des Kunststoffbehältnisses (10) über dessen Mündungsrand (10d) hinweg erstreckt.

14. Verfahren zum Sterilisieren von Kunststoffvorformlingen (10), wobei die Kunststoffvorformlinge (10) entlang eines vorgegebenen Pfades transportiert und während dieses Transports ein Innenraum dieser Kunststoffvorformlinge (10) von einer Vielzahl von Beaufschlagungseinrichtungen (4) mit einem fließfähigen Sterilisationsmittel beaufschlagt wird,
**dadurch gekennzeichnet, dass**
das fließfähige Medium unter einem vorgegebenen von 0° verschiedenen Winkel α gegenüber einer Längsrichtung (L) der Kunststoffvorformlinge (10) in diese eingegeben wird und/oder die Längsrichtung (LB) der Beaufschlagungseinrichtungen (4) gegenüber den Längsrichtungen (L) der Kunststoffvorformlinge (10) während der Beaufschlagung versetzt angeordnet ist, wobei zwischen den Mündungen der Kunststoffvorformlinge (10) und den Beaufschlagungseinrichtungen (4) ein sich in der Transportrichtung der Kunststoffvorformlinge erstreckender Spalt (8) ausgebildet ist, und weiter wobei die Höhe des Spalts (8) zwischen den Kunststoffvorformlingen (10) und den Beaufschlagungseinrichtungen (4) einstellbar ist.
